# EUROPÄISCHE PATENTSCHRIFT

(11) **EP 0 656 895 B1**
(45) Veröffentlichungstag und Bekanntmachung des Hinweises auf die Patenterteilung: **09.01.2002**
(21) Anmeldenummer: 94918747.0
(22) Anmeldetag: 28.06.1994
(51) Int. Cl.: C07D 311/96, C07D 493/10, C08K 5/00

(54) **Photochrome Spiropyran Verbindungen**
Photochromic spiropyran compounds
Composés de spiropyranne photochromiques

(30) Priorität: 28.06.1993 DE 4321485
(43) Veröffentlichungstag der Anmeldung: 14.06.1995
(73) Patentinhaber: Optische Werke G. Rodenstock, 80469 München (DE)
(72) Erfinder: MELZIG, Manfred, D-82234 Wessling (DE); ZINNER, Herbert, D-93080 Pentling (DE)
(74) Vertreter: Münich, Wilhelm, Dr.
(86) Internationale Anmeldenummer: DE9400743
(87) Internationale Veröffentlichungsnummer: WO9500504

(56) Entgegenhaltungen:
- EP-A- 0 401 958
- EP-A- 0 625 518
- WO-A-91/00861
- WO-A-93/10112

## Beschreibung

Die Erfindung bezieht sich auf photochrome Spiro- oder Dispiropyrane, insbesondere zum Einfärben von optischen Elementen aus einem Kunststoffmaterial.

Pyrane sind eine seit langem bekannte und gut untersuchte Klasse von photochromen Farbstoffen. So haben beispielsweise die in der US-PS 5 066 818 oder der US-PS 4 818 096 beschriebenen Diphenyl-Naphthopyrane recht gute Gebrauchseigenschaften.

Die in diesen Druckschriften beschriebenen Moleküle haben jedoch in verschiedenen Kunststoffen, insbesondere bei erhöhten Temperaturen, wie sie beispielsweise bei dem Färbe- und Entspiegelungsvorgang photochromer Brillengläser auftreten, eine hohe Mobilität. Diese Mobilität führt beispielsweise dann, wenn Brillengläser oder andere photochrom einzufärbende Gegenstände zur Herstellung neutral brauner oder grauer Einfärbungen sowohl mit Pyranen als auch mit photochromen Spironaphthoxazinen eingefärbt werden (vgl. auch EP-A-0 397 803), zu Schwierigkeiten:

Die Pyrane wandern bei der gemeinsamen Färbung von der Oberfläche her wesentlich schneller und damit tiefer in das Polymer als die Oxazine. Dort werden sie mit wesentlich weniger UV-Strahlung als die näher an der Oberfläche befindlichen Oxazine beaufschlagt, da diese die UV-Strahlung bereits weitgehend absorbieren. Damit ist die durch die Pyrane verursachte Einfärbung bezogen auf die eingebrachte Menge wesentlich geringer als die Einfärbung durch die Oxazine, so daß die Erzielung einer neutral braunen oder grauen Einfärbung schwierig ist.

Andererseits wandern die Pyrane bei der Entspiegelung, die üblicherweise bei einer Substrattemperatur von mehr als 80°C durchgeführt wird, wesentlich schneller an die Oberfläche als die Oxazine und führen dort zu Schichtablösungen.

Die in der US-PS-4 818 096 ebenfalls beschriebenen Adamantanospiropyrane haben zwar eine geringere Mobilität als Diphenyl-Naphthopyrane, ihre Aufhellgeschwindigkeit ist aber zu gering. Darüberhinaus liefern diese Farbstoffe nur gelbe Farbtöne.

Das Dokument EP-A-0 401 958 beschreibt photochrome Benzospiropyrane und Naphthospiropyrane, die substituiert sein können und in deren Strukturen die Reste in der 2-Stellung eines 2H-Pyranrings jeweils eine Alkylgruppe oder zusammen eine Norbornyliden- oder Bicyclo(3.3.1)9-nonylidengruppe bilden.

Das Dokument WO-A-91 00861 beschreibt photochrome Benzospiropyrane und Naphthospiropyrane, die substituiert sein können und in deren Strukturen die Reste in der 2-Stellung eines 2H-Pyranrings zusammen eine Norcamphergruppe oder ein alkylsubstituiertes Homolog davon oder eine Tricyclodecangruppe oder ein alkylsubstituiertes Derivat davon bilden.

Das Dokument WO-A-93 10112 beschreibt photochrome Naphthopyrane, die substituiert sein können und in deren Strukturen die Reste in der 2-Stellung eines 2H-Pyranrings jeweils aus einer Phenylgruppe bestehen, die substituiert sein kann.

Das aus einer prioritätsälteren, nicht vorveröffentlichten Anmeldung hervorgegangene Dokument EP-A-0 625 518 beschreibt photochrome Spiropyrane, die substituiert sein können und in deren Strukturen die Reste in der 2-Stellung eines 2H-Pyranrings zur Bildung eines fünfgliedrigen Rings miteinander verbunden sind, der in der Weise substituiert sein kann, daß ein Spiropyran der folgenden Struktur entsteht:

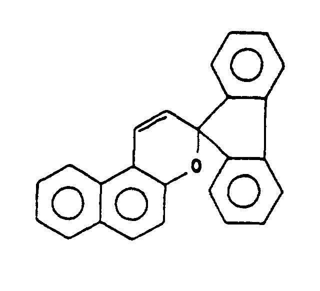

Der Erfindung liegt die Aufgabe zugrunde, photochrome Verbindungen anzugeben, die die guten Eigenschaften der beispielsweise aus der US-PS-5 066 818 beschriebenen Diphenyl-Naphthopyrane bei deutlich verringerter Mobilität in den insbesondere für Brillengläser verwendeten Kunststoffmaterialien haben.

Eine erfindungsgemäße Lösung dieser Aufgabe ist im Patentanspruch 1 angegeben. Weiterbildungen der Erfindung sind Gegenstand der Unteransprüche.

Erfindungsgemäß sind bei einem photochromen 2H-Pyran die aromatischen Reste in 2-Stellung chemisch starr zu einem Spiropyran verbunden Durch die starre Verbindung beispielsweise der beiden Phenylringe, die bei den in der US-PS-5 066 818 beschriebenen Molekülen gegeneinander leicht beweglich sind, wird die Diffusionsgeschwindigkeit der Moleküle drastisch reduziert.

Die Verbindung kann auf die verschiedensten Arten erfolgen, beispielsweise derart, daß dadurch ein Fluoren, Dibenzosuberon, Anthron, Xanthen oder Thioxanthen entsteht. Nachfolgend ist eine Strukturformel für die aus der US-PS-5 066 818 bekannten Pyrane dargestellt, wenn die Verbindung über -CO-zum Anthron erfolgt. 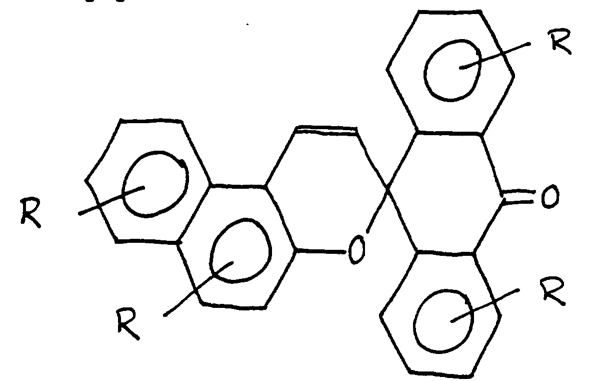 Alternativ kann die Verknüpfung durch 1 bis 3 CH₂-Brücken hergestellt werden. Das photochrome Pyran kann ein Diphenyl(naphtho)pyran oder ein substituiertes Dinaphthyloder Phenylnaphthylderivat sein. Der gleiche Effekt tritt ein, wenn der in 3-Stellung des Naphthalinsystems befindliche Substituent, beispielsweise Acetoxy- oder Benzoyloxy- durch die in der DE-A-38 14 631 beschriebene Oxadiazolgruppe ersetzt wird.

Geht man bei der Herstellung der photochromen Verbindungen von Dihydroxyverbindungen des Naphthalins, Phenanthrens etc. aus, so erhält man Dispiropyrane. Diese sind aufgrund ihres erheblich größeren sterischen Anspruchs ebenfalls weniger migrationsfreudig.

Darüberhinaus lassen sich durch die Umsetzung von Hydroxylgruppen in nicht äquivalenten Stellungen, beispielsweise im 1,6- oder 1,3-Dihydroxynaphthalin, mit einem Acetylid oder durch Umsetzung mit verschiedenen Acetyliden photochrome Moleküle synthetisieren, die zwei (oder mehr) unterschiedlich in Farbe und Kinetik reagierende photochrome Teilsysteme tragen, wie dies in einer am gleichen Tag eingereichten Anmeldung desselben Anmelders beschrieben ist. Es ist auch möglich, mehrere oder alle beschriebenen Lösungswege in einer Verbindung zu realisieren, indem man beispielsweise 3,8-Dihydroxynaphthoe-(2)-säure zum entsprechenden 2-Diazolderivat umsetzt und dieses zunächst in 8-Stellung mit einem Anthrolacetylid, dann in 3-Stellung mit einem Fluorenolacetylid umsetzt.

Da die Messung der tatsächlichen Diffusionsgeschwinddigkeit photochromer organischer Moleküle in Kunststoffmaterialien außerordentlich aufwendig und kompliziert ist, wird eine indirekte Bestimmungsmethode gewählt. Unter Wärmeeinwirkung tritt eine Rückwanderung der photochromen Moleküle in einem, beispielsweise entsprechend der DE 3345639 eingefärbten Brillenglas aus Polydiethylenglykolbisallylcarbonat ( Handelsname CR 39 ) an die Oberfläche auf. Diese Wärmeeinwirkung ist beispielsweise bei der Entspiegelung derartiger Brillengläser unvermeidlich und tritt auch im üblichen Gebrauch von Brillen auf, z.B. im Sommer in einem Pkw-Handschuhfach. Die rückwandernden photochromen Moleküle führen an der Grenzfläche Kunststoffmaterial/Entspiegelungsschicht zu Defekten, sei es durch Ablösungserscheinungen der Schicht oder durch Farbstoffagglomeration. Letztere ist mit den bloßen Auge als Fleckenbildung unter Lichtanregung erkennbar.

Es wurden entsprechend der nachfolgenden Versuchsbeschreibung 12 erfindungsgemäße Spiropyrane und 5 bekannte Spiropyrane, wie in Tabelle 1 angegeben, hergestellt und untersucht.
a) Herstellung eines Carbinols für Beispiel 1 : 39,6 g ( 0.22 mol ) 9-Fluorenon werden in 120 ml trockenem Dimethylsulfoxid gelöst, zu der neongelben Lösung werden unter Rühren 20,3 g ( 0.22 mol ) Lithiumacetylid--Ethylendiamin-Komplex zugetropft. Die Lösung färbt sich allmählich dunkelgelb. Die Mischung wird bei 25° C noch 16 h gerührt, dann auf 400 g zerstoßenes Eis gegossen und mit verd.HCl sauer eingestellt. Der sich beim Eintrag der Lösung in Eis bildende gelblich-weiße Niederschlag ballt sich bei der Neutralisation zusammen. Die Suspension wird mit Ether solange ausgeschüttelt, bis sich der an der Phasengrenze absetzende Niederschlag völlig im Ether aufgelöst hat. Die vereinigten Etherextrakte werden mit Na₂SO₄ getrocknet und filtriert. Dem Filtrat wird der Ether am Rotationsverdampfer entzogen. Zurück bleiben 21 g eines gelben Öles, das für die weitere Verarbeitung ausreichend rein ist.
b) Umsetzung zum Pyran :
   Das unter a) erhaltene Produkt wird zusammen mit 20 g (0.1 mol ) 3-Acetyl-2-naphthol in 1600 ml Toluol zunächst 8 h bei Raumtemperatur gerührt, dann 2 h unter Rückfluß gekocht. Die noch warme Lösung wird durch Ausschütteln mit Na₂CO₃-Lösung von noch vorhandenem Ausgangsprodukt befreit. Die org.Phase wird mit Na₂SO₄ getrocknet, filtriert, auf ca. 70 ml eingeengt und mit Tolwol an Al₂O₃ chromatographiert. Die vorweglaufende gelbe Fraktion wird verworfen, die anschließende rot-orange-photochrome Fraktion gesammelt und zur Trocknung eingeengt. Das erhaltene Produkt wird aus Ether/Hexan umkristallisiert. Mittels NMR-Spektrum wird die erhaltene Substanz als Spiro(fluoren-9,2'-(2H)-naphtho (2,3-b)pyran ) identifiziert.

Die Herstellung der Beispiele 2-12 erfolgt in analoger Weise mit den entsprechenden Ketonen bzw. substituierten Naphtholen als Ausgangsverbindungen. Die 5 Vergleichsbeispiele werden nach den Originalvorschriften in den Patentveröffentlichungen synthetisiert.
c) Färbung :
   Mit den erfindungsgemäßen Verbindungen sowie den Vergleichssubstanzen nach dem Stand der Technik werden im Handel erhältliche Nullgläser aus Polydiethylenglykolbisallylcarbonat ( * 71 mm, Mittendicke ca. 2 mm ) mittels Lacktechnik in bekannter Weise ( DE 33 45 639 ) konvexseitig photochrom eingefärbt. Die Farbstoffkonzentration im spritzfertigen Lack beträgt 4%, die Färbung wird bei 160° C über 90 min durchgeführt. Je 10 Gläser werden mit einer Breitbandentspiegelung, je 10 weitere zunächst mit einer organischen Hartschicht auf Polysiloxanbasis und anschlieβend mit einer Superentspiegelung mit Clean-Effekt versehen. Diese Testgläser werden 6 h einer Temperatur von 65° C ausgesetzt und anschließend optisch beurteilt. Bei Fehlerfreiheit wird der Test bis zu maximal 5 mal wiederholt. Die Ergebnisse sind in Tabelle 2 und 3 dargestellt.

**Tabelle 1 :**

| Substitutionsschema erfindungsgemäßer und bekannter photochromer Verbindungen | | | | | |
|---|---|---|---|---|---|
| Beispiel | R | R' | R'' | R''' | X |
| 1 | H | H | OCOCH₃ | H | - |
| 2 | H | H | H | H | - |
| 3 | H | H | H | H | CH₂ |
| 4 | H | H | H | H | CH₂ - CH₂ |
| 5 | H | H | H | H | O |
| 6 | H | H | H | H | S |
| 7 | H | H | H | 2,3-benzo | - |
| 8 | OCH₃ | H | H | H | - |
| 9 | H | OCH₃ | H | H | - |
| 10 | H | H | H | 1-CH₃ | - |
| 11 | H | Br | H | 1-CH₃ | - |
| 12 | H | H | H | OCOC₆H₅ | - |

Vergleich (alle Vergleichsbeispiele besitzen über X nicht verbundene, also freie Arylreste)

| | | | | | |
|---|---|---|---|---|---|
| 1 | H | H | H | H | US 3 567 605 |
| 2 | OCH₃ | H | H | H | |
| 3 | H | OCH₃ | H | H | US 5 238 981 |
| 4 | H | H | OCOCH₃ | H | WO 92/09593 |
| 5 | H | H | H | 2-CH₃ | US 5 066 818 |

**Tabelle 2 :**

| Verhalten mit Breitbandentspiegelung; Ausfälle im x. Zyklus | | | | | |
|---|---|---|---|---|---|
| Beispiel | x= 1 | 2 | 3 | 4 | 5 |
| 1 | - | - | - | 1s | 2s |
| 2 | - | - | 1s | - | 1s |
| 3 | - | - | 2s | 2s | 1s/1f |
| 4 | - | 1s | 2s | 2s | 3s |
| 5 | - | - | 1s | 1s | 2s/1f |
| 6 | - | - | 1s | 2s | 1s |
| 8 | - | - | - | 2s | 1s |
| 10 | - | - | - | 1s | 1s/1f |
| 12 | - | - | - | - | 2s |

| Vergleich | | | | | |
|---|---|---|---|---|---|
| 1 | - | 2s/2f | 1s/4f | 1s | |
| 2 | 1s | 5s/1f | 2f | 1f | |
| 3 | - | 4s/2f | 2s/2f | | |

**Tabelle 3 :**

| Verhalten mit Hartschicht und Superbreitbandentspiegelung | | | | | |
|---|---|---|---|---|---|
| 1 | - | - | - | 1s | 1s |
| 2 | - | - | 1s | 2s | 1s |
| 3 | - | - | 3s | 1s | 1s |
| 4 | - | 1s | - | 2s/1f | 3s |
| 5 | - | - | 2s | 4s/1f | 2s |
| 7 | - | - | - | - | - |
| 9 | - | - | 1s | 3s | 3s |
| 11 | - | - | 1s | 1s | 2s/1f |

| Vergleich | | | | | |
|---|---|---|---|---|---|
| 4 | - | 2s/3f | 3s/1f | 1s | |
| 5 | - | 4s | 6s | | |

- Wertung :: s = Schichtablösung, f = Fleckigkeit beim Auftreten beider Fehler in einem Glas bei der Kontrolle wurde nur die Fleckigkeit bewertet

## Patentansprüche

1. Photochrome Spiro- oder Dispiropyrane in deren Struktur aromatische Reste in der 2-Stellung eines Diphenyl-2H-pyrans, das substituiert sein kann, Diphenyl-2H-naphthopyrans, substituierten Dinaphthyl-2H-pyrans oder substituierten Phenylnaphthyl-2H-pyrans chemisch starr miteinander verbunden sind, mit Ausnahme von Spiropyranen nach EP 0 625 518 A1 mit der Struktur

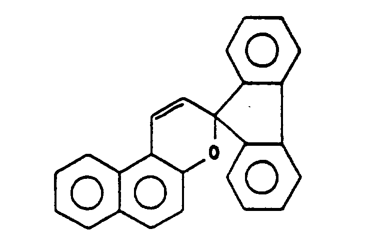

2. Photochrome Spiro- oder Dispiropyrane nach Anspruch 1, bei denen die aromatischen Reste Phenylreste sind.

3. Photochrome Spiro- oder Dispiropyrane nach Anspruch 1 oder 2, bei denen durch die Verbindung der aromatischen Reste ein Fluoren, Dibenzosuberon, Anthron, Xanthen oder Thioxanthen entstanden ist.

4. Photochrome Spiro- oder Dispiropyrane nach Anspruch 1 oder 2, bei denen die aromatischen Reste durch 1 bis 3 CH₂-Brücken miteinander verbunden sind.

5. Photochrome Dispiropyrane nach einem der Ansprüche 1 bis 4, deren photochrome Moleküle zwei unterschiedlich in Farbe und Kinetik reagierende photochrome Teilsysteme tragen.

6. Verwendung der photochromen Spiro- oder Dispiropyrane nach einem der Ansprüche 1 bis 5 zum Einfärben von optischen Elementen aus einem Kunststoffmaterial.

## Claims

1. Photochromic spiro- or dispiropyrans in the structure of which aromatic groups are bonded together chemically rigidly in the 2-position of a diphenyl-2H-pyran, which can be substituted, of diphenyl-2H-naphthopyran, of substituted dinaphthyl-2H-pyran or of substituted phenylnaphthyl-2H-pyran, with the exception of spiropyrans according to EP 0 625 518 A1, with the structure

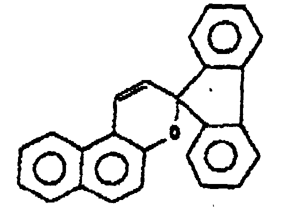

2. Photochromic spiro- or dispiropyrans according to claim 1, in the case of which the aromatic groups are phenyl groups.

3. Photochromic spiro- or dispiropyrans according to claim 1 or 2, in the case of which a fluorene, dibenzosuberone, anthrone, xanthene or thioxanthene is formed by means of the bonding of the aromatic groups.

4. Photochromic spiro- or dispiropyrans according to claim 1 or 2, in the case of which the aromatic groups are bonded together by means of 1 to 3 CH₂ bridges.

5. Photochromic dispiropyrans according to one of the claims 1 to 4, the photochromic molecules of which carry two photochromic part-systems which react differently in colour and in kinetics.

6. Use of the photochromic spiro- or dispiropyrans according to one of the claims 1 to 5 for pigmentation of optical elements made of a plastic material.

## Revendications

1. Spiro- ou dispiropyranes photochromes dans la structure desquels des résidus aromatiques sont liés entre eux de manière chimiquement rigide dans la position 2 d'un diphényl-2H-pyrane le cas échéant substitué, d'un diphényl-2H-naphtopyrane, d'un dinaphtyl-2H-pyrane substitué ou d'un phénylnaphtyl-2H-pyrane substitué, à l'exception des spiropyranes suivant EP 0 625 518 A1 ayant la structure suivante :

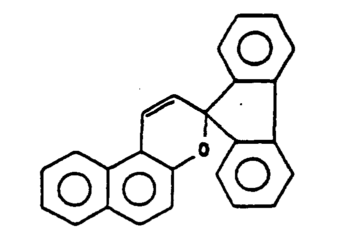

2. Spiro- ou dispiropyranes photochromes selon la revendication 1, dans lesquels les résidus aromatiques sont des résidus de phényle.

3. Spiro- ou dispiropyranes photochromes selon la revendication 1 ou la revendication 2, dans lesquels la liaison des résidus aromatiques a donné un fluorène, une dibenzosubérone, une anthrone, un xanthène ou un thioxanthène.

4. Spiro- ou dispiropyranes photochromes selon la revendication 1 ou la revendication 2, dans lesquels les résidus aromatiques sont liés entre eux par 1 à 3 ponts CH₂.

5. Dispiropyranes photochromes selon l'une des revendications 1 à 4, dont les molécules photochromes portent deux systèmes partiels qui réagissent différemment du point de vue de la couleur et de la cinétique.

6. Utilisation des spiro- ou dispiropyranes photochromes selon l'une des revendications 1 à 5 pour colorer des éléments optiques faits d'un matériau plastique.
